Europäisches Patentamt

(19) European Patent Office

Office européen des brevets

(11) Publication number: **0 043 359**
**A2**

(12) # EUROPEAN PATENT APPLICATION

(21) Application number: **81850116.5**

(22) Date of filing: **29.06.81**

(51) Int. Cl.³: **G 01 N 33/54**
**G 01 N 33/58**

(30) Priority: **30.06.80 SE 8004815**

(43) Date of publication of application:
**06.01.82 Bulletin 82/1**

(84) Designated Contracting States:
**BE DE FR GB NL SE**

(71) Applicant: **PHARMACIA DIAGNOSTICS AB**
**Rapsgatan 7**
**S-751 03 Uppsala 1(SE)**

(72) Inventor: **Cerven, Erik**
**26 Blodstensvägen**
**S-752 44 Uppsala(SE)**

(74) Representative: **Onn, Thorsten et al,**
**AB STOCKHOLMS PATENTBYRA Box 3129**
**S-103 62 Stockholm(SE)**

(54) **Determination of terminal saccharide groups in glycoprotein.**

(57) A method of determining qualitatively and/or quantitatively terminal saccharide groups in glycoproteins from body liquids having a microheterogeneity in the saccharide group or groups. The method comprises three steps : 1) Binding of glycoprotein antibodies to a solid phase: 2) Binding of glycoprotein from body liquid and from standard solutions with a defined composition of terminal saccharide groups to the product from step 1. 3) Binding of labeled lectin to the complex of antiglycoprotein, glycoprotein and solid phase from step 2. The amount of bound labeled lectin can then be measured in the product from step 3 or the amount of unbound marked lectin in the supernatant and possible wasing solutions can be measured. This amount is correlated to the amount of terminal saccharide groups in the glycoprotein and on the basis of this the amount of a glycoprotein component with a special composition of terminal saccharide groups can also be determined. The method can be used in medical diagnostics.

FIG.1

SERUM DESIALOTRANSFERRIN
(O, per cent OF TOTAL TRANSFERRIN)

## Determination of terminal saccharide groups in glycoprotein

This invention relates to determination of various terminal saccharide groups in glycoprotein components from body liquid, said glycoprotein showing microheterogeneity in the saccharide group or groups. The term glycoprotein is intended to include glycopeptides, oligosaccharides and polysaccharides.

The invention is especially intended for use with quantitative determination of terminal saccharide groups in glycoprotein components in body liquid from human beings, e.g. plasma, serum or saliva even if body liquid from animals of course can be analyzed. The invention can also be utilized for qualitative determination of said saccharide groups. By saccharide groups sugar groups are intended.

Glycoproteins consist of at least one polypeptide portion and at least one carbohydrate portion. Glycoproteins are very frequent in biological tissues and liquids. Various $\gamma$-globulins (i.a. immunoglobulins), fibrinogen, transferrin, prothrombin, ceruloplasmin, haptoglobin, hemoglobin, carcinoembryonic antigen (CEA), $\alpha$-fetoprotein, orosmucoid, fetuin, ferritin, C-reactive protein, prostatic acid phosphatase, mucoproteins, certain enzymes, serum $\alpha_1$-proteinase inhibitor etc. can be mentioned here.

Certain known medically, clinically defineable states are accompanied by a changed microheterogeneity in the saccharide groups in glycoprotein from body liquid, i.e. one or more glycoprotein components with a certain composition of terminal saccharide groups are included in an abnormal amount. Such a clinical state is alcoholism. It is known that a prolonged, high alcohol consumption is accompanied by a selective, mostly marked increase of the amount of a transferrin component in serum, which component has an isoelectric pH-value (pI) of 5.7 and is normally a minor portion (about 3 %) of the total transferrin content. The principal transferrin component

has a pI-value of 5.4. An increased amount of the transferrin component having a pI-value of 5.7 is consequently
an indication of a prolonged, high alcohol consumption. The charge
of pI being dependent of the amount of terminat sialic acid residues.

At present such a transferrin abnormality is established by a method including isoelectric focusing followed by immunofixation and quantitative densitometry. As
this method is time-consuming and requires special equipment it is not suitable for clinical use on a large scale
(Subst Alcohol Actions Misuse 1 (1980), pages 247-252).

Other clinical conditions have also been correlated
to small changes in the carbohydrate portion of glycoproteins. See e.g. Int J Cancer Res 22 (1978), pages 515-20
and Biochem Biophys Acta 458 (1976), pages 355-373. These
articles discuss microheterogeneity in the carbohydrate
portion of CEA and α-fetoprotein, respectively, as a consequence of various diseases.

It is consequently an object of the invention to
develop a simple and sensitive method for qualitative
and/or quantitative determination of terminal saccharide
groups in microheterogeneous glycoproteins which method is
suitable for routine use on a large scale.

According to the invention this object is acchieved
by a method characterized by the following steps:

1) antibodies with a specificity to the glycoprotein to be determined are bound to a solid phase, an
immunoadsorbent being formed;

2) glycoproteins from samples of body liquid or
from different standard solutions, which solutions each
contain glycoprotein with a defined composition of terminal saccharide groups, are bound to the antibody of the immunad-
sorbent, an immunoadsorbent glycoprotein complex being formed;

3) labeled lectin or lectin, which can be labeled,
is incubated with the immunoadsorbent glycoprotein complex from
step 2, in which complex one or more terminal saccharide groups in
the glycoprotein has optionally been modified, which lectin during
incubation is bound to the terminal saccharide group to be determined
in the microheterogenic glycoprotein of the complex, after which the amount of lectin bound by the complex.from step 2 or the amount of lectin unbound by this complex is determined in a way known per se.

The method of the invention can be given the rational name radio-lectin immunoassay (RLIA).

The antibodies used in the first step of this method, i.e. immunoglobulins having specificity to a human glycoprotein to be analyzed, are prepared by methods based on technique previously described. Such methods include e.g. that the glycoprotein to be analyzed is injected into an animal where it will give rise to antibodies, i.e. immunoglobulins with the desired specificity, which are separated and purified in a way known per se. Many such antibody preparations are commercially available.

In the first step according to the invention such immunoglobulins are bound to a solid phase by any suitable known technique. The invention is then not restricted to any special technique now known. Moreover, such methods can probably also be used as can possibly be developed in the future. In this connection a phase, which can be easily separated from a liquid phase making it possible to change and renew the liquid phase, is intended by a solid phase. The product obtained when the immunoglobulins are bound to a solid phase is called immunoadsorbent.

This method of rendering the antibodies insoluble can be carried out in various manners, e.g. by covalent binding of antibodies to a solid matrix, such as particles of agarose (e.g. Sepharose®, Pharmacia Fine Chemicals AB, Sweden), cross-linked dextran (e.g. Sephadex®, Pharmacia Fine Chemicals AB, Sweden) or cellulose. At covalent binding to hydroxyl group containing polymers such as Sepharose® gel the gel is preferably activated by means of cyanogen bromide under alkaline conditions before the addition of antibodies (see e.g. US patent 3 645 852). Also cross conjugation, incorporation in polyacrylamide gels or physical adsorption to activated carbon, glass or plastic surfaces, can be utilized for the insolubilization. Due to the great number of methods available for insolubilization the reaction time, pH, temperature and other variables can be allowed to vary within wide limits. Of course each method has its permitted ranges of the variables. The essential thing is that the antibodies after the insolubilization retain completely or partly their antigen binding ability.

The second step according to the invention comprising binding of glycoprotein to the immunoadsorbent can be carried out in such a way that the immunoadsorbent is incubated with the glycoprotein in solution or with the body liquid to be  investigated. The body liquid can be diluted, if necessary.

The incubation means that the glycoprotein is bonded to the antibody of the immunoadsorbent due to a biospecific affinity. The incubation can be carried out at a temperature of $-10^{\circ}$ to $+50^{\circ}$C conveniently $10-40^{\circ}$C and preferably $20-40^{\circ}$C, and at a pH-value of 3-9, preferably 6-8. These conditions are not especially critical and suitable conditions can be easily established by one skilled in the art. The incubation time can vary from some minutes, e.g. 5 minutes, to a couple of hours or longer. Usually a time of maximum 2 hours· is enough.

After the immunoadsorbent has been reacted with the glycoprotein to be investigated the solid phase may be washed.

In the resulting complex terminal saccharide groups in the glycoprotein can be modified in respect of transferrin, e.g. by incubation with galactosidase, to N-acetyl-glycosamide, GlNac, and thereafter with N-acetyl glycosaminidase to mannose.

The third step according to the invention comprising binding of lectin to the immunoadsorbent antigen complex from step 2 is carried out in a way known per se. The lectin then used is capable of being bound biospecifically to the terminal saccharide group which is to be determined in the microheterogenic glycoprotein.
The gel from step 2 is preferably incubated at a temperature of about 37°C for about 10-90 min. after which unbound lectin is removed. The incubation conditions are not specifically critical but about the same ranges of temperature, pH and time indicated above for step 2 can be used. The amount of lectin bound or, alternatively, not bound to terminal saccharide groups is then measured in a suitable manner. At labeling with an isotop a liquid scintillation counter or $\gamma$-counter can be used.

One advantage of the method of the invention is that very small amounts of glycoprotein are enough for utilization of the invention, as amounts below 1 nmol of terminal saccharide groups can be determined both quantitatively and qualitatively. Moreover, a resolving power of 30 pmoles saccharide has been achieved, which value can probably be improved if the test conditions are optimized.

Glycoproteins which can be determined according to the invention are such as a) give rise to formation of antibodies when injected into an animal, whereby an antibody fraction formed can be separated and purified, and b) show microheterogeneity in the saccharide group or groups. It is then intended by microheterogeneity that glycoproteins which are bound to the same antibody preparation can show different terminal saccharide groups in the saccharide portion. The terminal saccharide groups can e.g. consist of sialic acid, galactose, or mannose. Moreover, the terminal saccharide groups of these glycoproteins should not be antigen determinants, i.e. antibodies formed with a specificity to the glycoprotein should not react with terminal saccharide groups in the glycoprotein.

An essential characteristic feature of the invention is the use of lectins for quantitative and/or qualitative determination of different terminal saccharide groups in a microheterogenic glycoprotein.

By lectins proteins, optionally glycoproteins, are intended, which can be obtained from animal or plant sources, and are capable of binding to specific saccharide groups. Examples of such lectins are galactose binding lectin from Crotalaria juncea (see Ersson, B., Afpberg, K. and Porath, J., Biochim. Biophys. Acta, volume 310 (1973), pages 446-452, and Ersson, B., Biochim. Biophys. Acta, volume 494 (1977), pages 51-60), a podded plant, and a sialic acid binding lectin from the horseshoe crab, Limulus polyphemus.

According to the invention labeled lectin or lectin, which can be labeled, is used in order that the terminal saccharide groups in the glycoprotein might be determined qualitatively and/or quantitatively. Isotop labeling, e.g. by means of iodine $^{125}I$ or $^{131}I$ can be used with advantage. Also other labeling methods which are known and frequently used in immunological and similar methods can be used. As examples labeling with enzymes or fluorescent dyestuffs, such as fluorescein or rhodamine, or diazotized dyestuffs, such as Evans blue, can be mentioned.

By means of labeled lectin or lectin, which can be labeled, the content of the terminal saccharide groups of a microheterogenic glycoprotein, which are capable of being bound to the lectin, can be determined in a sample. This can be carried out by comparing the amount of lectin which is bound to the immunoadsorbent glycoprotein complex of a sample with the amount which is bound to the corresponding complex obtained if the immunoadsorbent instead of being reacted with a sample is reacted with standard solutions containing known contents of the glycoprotein with the terminal saccharide group which is to be determined quantitatively.

Alternatively the amount of unbound lectin remaining in the supernatant and possibly in washing solutions can be measured. At isotop labeling of the lectin a liquid scintillation counter or $\gamma$-counter is preferably used for determination of the amount of bound or free lectin. At labeling with fluorescent dyestuffs microspectrofluoro-metry can e.g. be utilized for quantitative determination. By using different types of lectin bound to different terminal saccharide groups it is also possible to determine which terminal saccharide groups are included in a glycoprotein and possibly also in which ratio these are included.

As to the conditions used when carrying out the different steps according to the invention these are not especially critical, as stated above. Conditions frequently used in immunological processes can be used.

As stated above the method of the invention can be utilized with advantage at determination of a certain transferrin component occurring in an increased amount in serum from persons with a prolonged high consumption of alcohol.

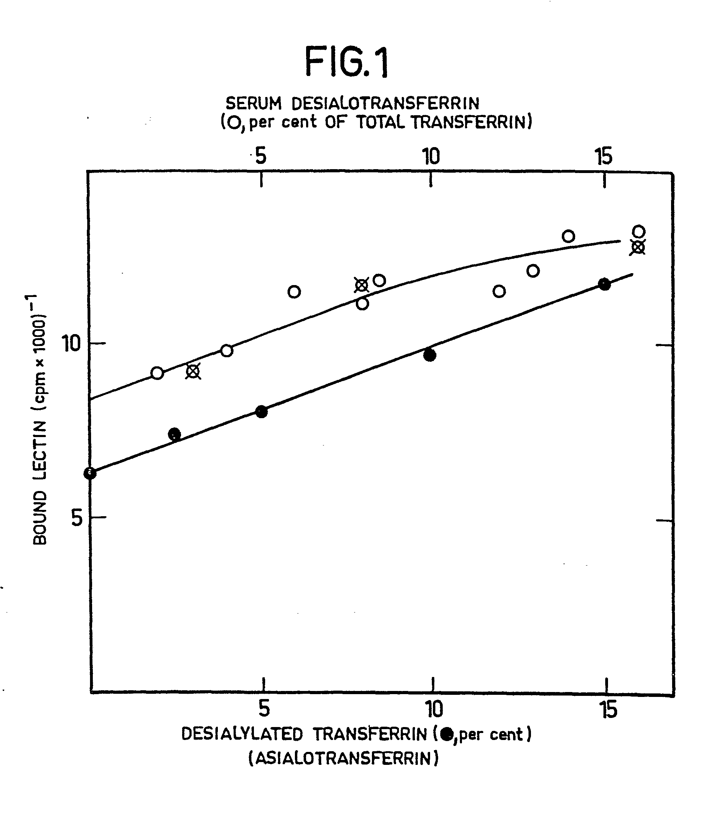

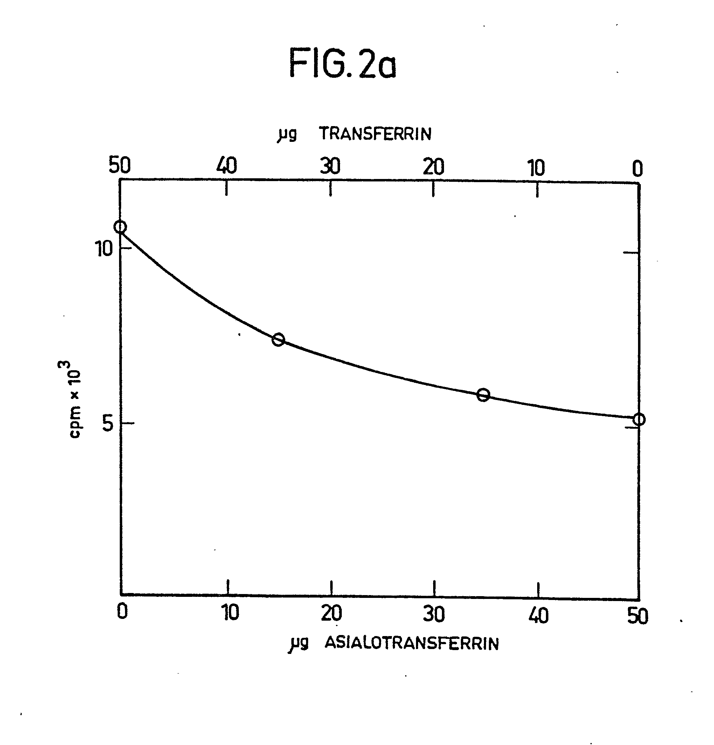

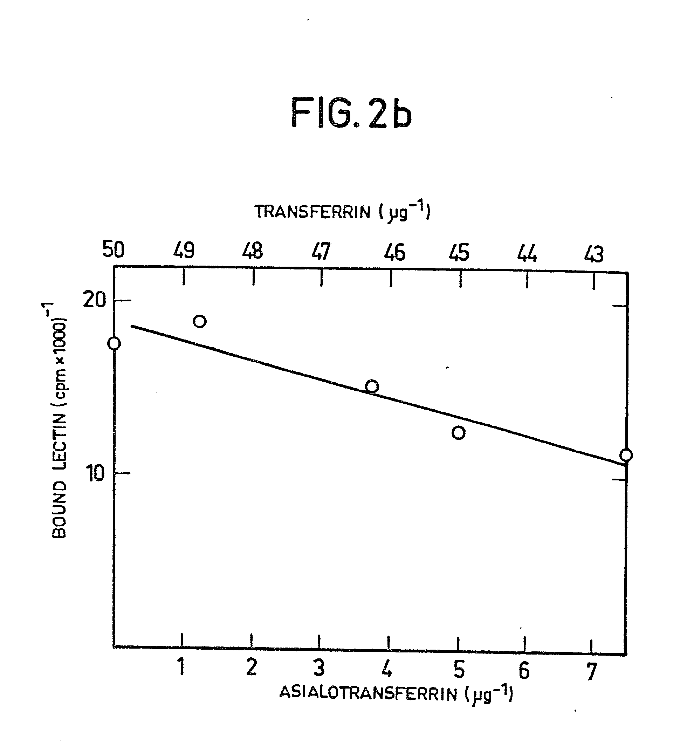

Therefore the invention is described in the following examples more in detail with reference to such a transferrin component determination. The results of these examples are compiled in diagrams on the enclosed drawings, Fig. 1 showing a diagram of analysis results obtained when using labeled Crotalaria lectin and Figs. 2A and B showing diagrams of analysis results obtained by using labeled Limulus lectin.

Example 1: Determination of asialotransferrin by means of Crotalaria lectin.

A. Preparation of immunoadsorbent

25 ml of Sepharose ® 4 B washed with distilled water are suspended in 25 ml of re-distilled water, and cooled to $20^{o}C$ and transferred to a beaker with magnetic stirring. 5 g of cyanogen bromide are added and the pH-value is adjusted to and maintained at 11 by addition of ice-cold sodium hydroxide solution (2 moles/l). The temperature is kept at $20^{o}C$ by means of crushed ice and after almost complete titration (after about 15 min) the gel is washed in an ice-cooled Büchner funnel with ice-cooled sodium phosphate buffer (0.07 moles/l phosphate,

pH 8.0). This gel is added to an equal amount of antitransferrin solution (Dako., Copenhagen, Denmark) (10 mg/ml) at room temperature, and is allowed to stand at room temperature with magnetic stirring for 2 h. Ethanolamine-HCl (1 mole/1, pH 8.0) is then added (5 ml/ml of gel) and the mixture is incubated with stirring for additional 2 h. The buffer is exchanged by washing and centrifugation to 0.05 moles/1 of sodium phosphate buffer, pH 7.5, containing 1 mole/1 sodium chloride. The gel is stored at 4°C in an equal volume of this buffer.

B. Formation of immunoadsorbent-glycoprotein complex

Standard solutions containing various percentages of asialotransferrin and transferrin are prepared by transferring 0.069 U agarose-bound neuraminidase from Dactylium dendroides (Sigma, S:t Louis, Mo, USA) having an activity of 44 U/g of agarose (1 U releases 1 μmole/min of sialic acid from AcNeu-lactose at pH 5.0 and 37°C) to sodium phosphate buffer (0.01 mole/1, pH 6.3). In this buffer 1 mg of human transferrin (AB Kabi, Sweden) is dissolved which is added to the agarose to a total volume of 1 ml and is incubated with shaking for 30 min at 37°C. The agarose is pelleted at the bottom of the tube and asiolotransferrin is transferred to another tube. 1 mg of untreated transferrin is dissolved in the same buffer and standard solutions are prepared by mixing the asialotransferrin solution and the transferrin solution in various proportions.

10 μl of antitransferrin-gel from A) in a total volume of 20 μl of phosphate buffer (0.05 moles/1, pH 7.5) containing sodium chloride (1 mole/1) are introduced into ice-cooled plastic centrifuge tubes. Transferrin in the sample and standard solutions is in this example and example 2 in excess of the transferrin during incubation. This gel is thereafter incubated with shaking at 37°C for 10 min with 1) different standard solutions of 50 μg of transferrin asialotransferrin of different composition in 50 μl of phosphate buffer (0,01 mole/1, pH 6,3) and 2) with samples of 100 μl of serum from different persons after a preceding additon of 100 μl sodium phosphate (0.05 moles/1) - citrate (0.02 moles/1), pH 7.2. The gel is
then freed by washing from soluble transferrin.

C) Formation of immunoadsorbent glycoprotein lectin
complex

The gel is then incubated with 50-100 µg of $^{125}$I-
-labeled Crotalaria lectin for 10 min in a thermostated
shaking device at 37$^{\circ}$C. Unbound lectin is removed from
the pelleted gel, after which the latter is suspended in
a buffer of 0.5 ml, is transferred to a scintillation
container and counted in the presence of scintillation
liquid. (Aquasol®, NEN Chemicals, Germany).

Crotalaria lectin had been labeled in the following
way: Crotalaria lectin was solubilized in sodium phosphate buffer (0.07 moles/1, pH 8.0) to a concentration
of 10 g/1 and the solution was cooled with ice. 0.09
nmoles of Bolton-Hunter reagent (NEN Chemicals, Germany
(2200 Ci/mmole) were evaporated in nitrogen from a solu-
tion in 20 µl of anhydrous benzene in a glass centrifuge
tube.

About half the benzene volume was evaporated at room
temperature for 10 min and the residue for 20 min after
placing the tube in an ice bath.

5 µl of the Crotalaria lectin solution (50 µg of
lectin) were thereafter added from an ice-cooled micro-
pipette and it was left for 1 hour at 0$^{\circ}$C and then for 3
hours at 4$^{\circ}$C. 1 mg of the Crotalaria lectin solution was
then added and the macromolecular material was eluted
from a Sephadex® G-25 column (PD-10, Pharmacia, Uppsala)
with a simultaneous buffer exhange to sodium phosphate
(0.05 moles/1, pH 7.5) containing sodium chloride (1
mole/1). Fractions of 1 ml were collected. The lectin
remained active for at least 4 months after storage at
4$^{\circ}$C in the latter buffer.

Example 2: Determination of sialic acid as terminal
group in transferrin.

The method in example 1 was repeated except for the
fact that $^{125}$I-labeled sialic acid binding lectin from Limulus polyphemus was used in C) instead of Crotalaria lectin. 0.1 ml of Na-EDTA (0.1 mole/1, pH 7.4) was first added to the immunoadsorbent transferrin complex prepared according to example 1A and 1B. Exchange of buffer to Tris-HCl (0.05 moles/1, pH 7.2) containing NaCl (0.15 moles/1) and $CaCl_2$ (0.1 mole/1) was then carried out by washing. About 20-25 μg of labeled Limulus lectin dissolved in 0.1 ml of the latter buffer were then added followed by three washes.

The Limulus lectin had been labeled as in example 1 except for the fact that labeling had been carried out at a lower concentration (1 g/1) and with a ratio of protein to Bolton-Hunter reagent being one tenth of that used in example 1. After labeling the Limulus lectin was diluted with an equally great volume of Na-EDTA (0.1 mole/1, pH 7.4) and transferred to a buffer of Tris-HCl (0.05 moles/1, pH 7.2), NaCl (0.15 moles/1) and $CaCl_2$ (0.1 mole/1).

The results from example 1 are shown in the diagram in Fig. 1. A molar ratio of transferrin to galactose binding lectin of about 1:1 has been used as presumably maximally only one lextin molecule can be bound to one of the galactose groups exposed in asialotransferrin. The measurements have been carried out in the range clinically interesting for investigation of persons' alcohol habits, which is 0-15 % of asialotransferrin based on total transferrin amount.

In Fig. 1 two curves are shown as obtained with RLIA of terminal galactose groups in 1) standard solutions containing 50 μg of transferrin with an indicated ratio of asialotransferrin (filled circles) or in 2) 100 μl of serum containing an indicated ratio of the transferrin component with a pI of 5.7 (unfilled circles). The standard ⊗ curve and three coordinates ⊗ in the serum transferrin curve are authentic while other coordinates in the serum transferrin curve 0 represent values obtained from another preparation of antitransferrin gel, a correction equivalent to each coordinate being inserted for the higher background value of lectin binding to the latter gel. The background binding of the serum incubations was 8-9000 cpm at the first incubation, and 12500 cpm at the second incubation. The lectin bound on the latter incubation occasion corresponds to 10-15 % of added lectin or 8-12 µg. About the same percentage of marked lectin is bound to partially hydrolyzed Sepharose ® containing an excess of terminal galactose residues, when the dilution ratio between marked and unmarked lectin is the same.

Table of Fig. 1

(incubation No. 2)

| Percentage of serum desialotransferrin with pI 5.7, determined by iso-electric focusing | Bound Crotalaria lectin using the claimed method (Radio lectin immunoassay) (cpm) |
|---|---|
| 2 | $13300 \pm 200$ |
| 4 | $13950 \pm 890$ |
| 6 | 15660 |
| 8 | $15340 \pm 890$ |
| 8 | $15980 \pm 1310$ |
| 12 | 15730 |
| 13 | $16260 \pm 1840$ |
| 14 | 17310 |
| 16 | $18690 \pm 160$ |

It is apparent from Fig. 1 that the standard curve in the range of 0 to 15 % of asialotransferrin has about the same inclination as the curve obtained with serum samples in the range of 2 to 16 % of the transferrin component with a pI 5.7. Moreover, such values lying in the lower left part of the serum curve, i.e. with a normal content of the transferrin component with pI 5.7, have been obtained with serum from persons having a low or normal alcohol consumption whereas values obtained with serum from persons with a high alcohol consumption lie in the upper right part of the serum curve, i.e. corresonding to a clearly increased amount of the transferrin component with pI 5.7.

The results from example 2 are shown in Figs. 2A and 2B. These diagrams show the results from RLIA of terminal sialic acid in transferrin with labeled Limulus lectin, 10 μl of antitransferrin Sepharose ® gel being incubated with 50 μg of transferrin containing untreated transferrin and completely desialylated asialotransferrin in indicated porportions, which gel has then been incubated with 25 μg of labeled Limulus lectin.

It is apparent from these diagrams that transferrin will bind a significantly larger amount of Limulus lectin than asialotransferrin does. On the other hand, the asialotransferrin antitransferrin complex binds significant amounts of Limulus lectin. This is probably due to the fact that sialyl groups are included in the antibodies and contribute to the binding of Limulus lectin.

Example 3: Determination of absolute concentration of asialotransferrin in serumstandards

A) CNBr-Activation of Sephacryl ®

2 g Sephacryl ® 400, allyl dextran cross-linked with N,N'-methylene bisacrylamide (Pharmacia Fine Chemicals AB, Sweden), was suspended in 40 ml potassium phosphate buffer (1,25 mol/l, pH 11,7) at 0°C, in an ice-bath.

2 g cyanogen bromide (Fluka AG, Switzerland) was weighed and dissolved in 40 ml distilled water at 20°C.

The cyanogen bromide solution was added gradually to the gel, using slow magnetic stirring. The components were allowed to react for 10 minutes at 0°C, with continued slow stirring.

The gel was then washed on a glass filter with 2 l ice-cold distilled water. Any free cyanogen bromide in the flask was eliminated by use of sodium hydroxide pellets.

The gel was acidified by washing with 250 ml acetic acid (0,01 mol/l).

The gel was then suspended in distilled water and ultrasonicated for between 1-15 minutes, until a homogenous preparation was obtained. The gel was sucked dry on a glassfilter using 100 ml acetone.

It was then transferred to a vacuum desiccator and allowed to dry under vacuum, until all traces of acetone smell had disappeared. The gel is stored dry at $4^{o}C$.

B) Coupling of antitransferrin to the activated gel

150 mg activated gel was added to a solution containing 1,25 ml antitransferrin (1,9 mg/ml) (DAKO, Denmark) and 1,5 ml (0,05 mol/l) BICIN buffer pH 8,3 (Sigma, US) containing 0,02 % $NaN_3$, 0,1 % Tween 20, 0,5 mol/l NaCl. This mixture was mixed thoroughly, then allowed to couple for 16 hours at $4^{o}C$, using end-over-end rotation.

Any excess active groups remaining on the gel after coupling were then blocked with 5 ml glycine (0,2 mol/l) dissolved in BICIN buffer pH 8,3).

The mixture was rotated end-over-end for 2 hours at room temperature.

Next the gel was washed four times each, with alternately acidic and basic pH buffer solutions. Buffers used: Acetate buffer (0,05 mol/l pH 4,0, 1 mol/l NaCl, 0,1 % Tween 20). Tris HCl buffer (0,05 mol/l pH 8,0, 1 mol/l NaCl, 0,02 % $NaN_3$, 0,1 % Tween 20).

The gel was finally washed with phosphate buffer pH 7,4 (0,04 mol/l $Na_2HPO_4 \cdot 2H_2O$, 0,008 mol/l $KH_2PO_4$, 0,16 mol/l NaCl, 0,008 mol/l $NaN_3$, 0,1 % Tween 20).

The gel was stored in the same phosphate buffer in a volume ratio of gel : buffer of 3:5.

C) Incubation of serumstandards with the immunoadsorbent

Standard solutions containing varying amounts of asialotransferrin were prepared by adding, to a serum pool with known quantitites of asialotransferrin and total transferrin, differing amounts of neuraminidase-treated transferrin (as described in an earlier example).

60 $\mu$l antitransferrin gel (from B) in a total volume of 100 $\mu$l phosphate buffer is placed in a series of test-tubes. The various serum standard solutions were diluted five times with saline buffer (= above described phosphate buffer). 100 ul of each was added, then incubated for 90 minutes at room temperature with shaking. This means that the available antigenbinding sites on the immunoadsorbent are in excess of the tranferrin during incubation.

Any unbound transferrin was washed from the gel with three washes of 2 ml phosphate buffer (0.05 ml/1 $Na_2HPO_4$, pH 7,5, 1 mol/1 NaCl, 0,1 % Tween 20, polyoxyethylene sorbitan monolaurate, Atlas Chemie, Germany).

D) Incubation with Crotalaria lectin

The gel was labelled with 20 $\mu$g $^{125}$I Crotalaria lectin in a 100 $\mu$l volume per tube. The tubes were then incubated, with shaking for 60 minutes at $37^{\circ}$C.

Unbound lectin was washed off with 4 x 2 ml phosphate buffer.

Radioactivity of the gel was then measured with a $\gamma$-counter for 2 minutes.

E) Results

The results from example 3 are presented in the following table.

| Concentration of serum-asialotransferrin (mg/ml) | Crotalaria Lectin Bound (cpm) |
|---|---|
| 0.10 | $14420 \pm 2020$ |
| 0.20 | $17720 \pm 815$ |
| 0.39 | $20165 \pm 403$ |
| 0.66 | $21922 \pm 1970$ |

Asialotransferrin concentrations of unknown samples may be determined by means of these standard samples. The total concentration of all types of tranferrin must be determined separately if percentage of asialotransferrin is to be calculated.

PATENT CLAIMS

1. A method of determining in a body liquid a) qualitatively and/or quantitatively terminal saccharide groups in glycoprotein components, which glycoprotein has microheterogeneity in the saccharide group or groups, and/or b) the content of such a glycoprotein component, characterized in that
1) antibodies with a specificity to the glycoprotein to be analyzed are bound to a solid phase, an immunoadsorbent being formed; that
2) the glycoprotein from samples of body liquid or from different standard solutions, which standardsolutions each contain glycoprotein with a defined composition of terminal saccharide groups, is bound to the antibody of the immunoadsorbent, an immunadsorbent glycoprotein complex being formed; and that
3) labeled lectin or lectin, which can be labeled, is incubated with the immunoadsorbent glycoprotein complex from step 2, in which complex one or more terminal saccharide groups in the glycoprotein have optionally been modified, which lectin during incubation is bound to the terminal saccharide group to be determined in the glycoprotein of the complex, after which the amount of lectin bound by the complex from step 2, and/or the amount of lectin not bound by this complex is determined in a way known per se.

2. The method of claim 1, characterized in that the glycoprotein is human transferrin and that the antibodies in step 1 are antitransferrin.

3. The method of claim 1 or 2, characterized in that the lectin is galactose binding lectin or   sialic acid binding lectin.

4. The method of any one of the preceding claims, characterized in that the solid phase is a gel.

5. The method of any one of claims 1-4, characterized in that the lectin is isotop-labeled.

6. The method of claim 5, characterized in that the lectin is labeled with iodine $^{125}I$.

7. The method of claim 5 or 6, characterized in that the amount of labeled lectin is measured by means of a liquid scintillation counter or $\gamma$-counter.

# FIG.1

SERUM DESIALOTRANSFERRIN
(O, per cent OF TOTAL TRANSFERRIN)

BOUND LECTIN (cpm × 1000)$^{-1}$

DESIALYLATED TRANSFERRIN (●, per cent)
(ASIALOTRANSFERRIN)

FIG.2a

μg   TRANSFERRIN

μg   ASIALOTRANSFERRIN

# FIG. 2b